# EUROPEAN PATENT SPECIFICATION

(11) **EP 2 499 983 B1**
(45) Date of publication and mention of the grant of the patent: **13.11.2013**
(21) Application number: 12160074.6
(22) Date of filing: 19.03.2012
(51) Int. Cl.: A61B 18/12, G01R 15/20, G01R 19/22, G01R 21/08, A61B 18/00, A61B 18/14

(54) **Isolated current sensor**
Isolierter Stromsensor
Capteur de courant isolé

(30) Priority: 17.03.2011 US 201113050770
(43) Date of publication of application: 19.09.2012
(73) Proprietor: Covidien LP, Mansfield, MA 02048 (US)
(72) Inventor: Smith, Robert B., Loveland, CO Colorado 80538 (US)
(74) Representative: HOFFMANN EITLE

(56) References cited:
- US-A- 4 094 320
- US-A- 4 196 734
- US-A- 6 142 992
- US-A1- 2007 161 979
- US-A1- 2010 179 536
- US-A1- 2011 054 460

## Description

### BACKGROUND

### Technical Field

The present disclosure relates to electrosurgical apparatuses, systems and methods. More particularly, the present disclosure is directed to an electrosurgical apparatus having an isolated current sensor.

### Background of Related Art

Energy-based tissue treatment is well known in the art. Various types of energy (e.g., electrical, ultrasonic, microwave, cryogenic, heat, laser, etc.) are applied to tissue to achieve a desired result. Electrosurgery involves application of high radio frequency electrical current, microwave energy or resistive heating to a surgical site to cut, ablate, coagulate or seal tissue.

In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned between the electrodes.

Bipolar electrosurgical techniques and instruments can be used to coagulate blood vessels or tissue, e.g., soft tissue structures, such as lung, brain and intestine. A surgeon can either cauterize, coagulate/desiccate and/or simply reduce or slow bleeding, by controlling the intensity, frequency and duration of the electrosurgical energy applied between the electrodes and through the tissue. In order to achieve one of these desired surgical effects without causing unwanted charring of tissue at the surgical site or causing collateral damage to adjacent tissue, e.g., thermal spread, it is necessary to control the output from the electrosurgical generator, e.g., power, waveform, voltage, current, pulse rate, etc.

In monopolar electrosurgery, the active electrode is typically a part of the surgical instrument held by the surgeon that is applied to the tissue to be treated. A patient return electrode is placed remotely from the active electrode to carry the current back to the generator and safely disperse current applied by the active electrode. The return electrodes usually have a large patient contact surface area to minimize heating at that site. Heating is caused by high current densities which directly depend on the surface area. A larger surface contact area results in lower localized heat intensity. Return electrodes are typically sized based on assumptions of the maximum current utilized during a particular surgical procedure and the duty cycle (i.e., the percentage of time the generator is on).

In addition to the above, US 6,142,992 A discloses a power supply for limiting power in electro-surgery. The input of a bridge rectifier is a transformer coil whose windings are wrapped around the electrode lines. Further, a current is sensed as an output current of a DC/DC converter. Furthermore, the voltage induced by the transformer coil is rectified, filtered, and fed back to a control input. Yet further US 4,094,320 A and US 4,196,734 A were found to disclose related subject-matter.

### SUMMARY

The mentioned drawbacks are solved by the subject-matter of the independent claims. Further preferred embodiments are defined in the dependent claims.

A method for electrosurgery for using the electrosurgical generator of the present invention is also contemplated by the present disclosure. The method includes: supplying at least one radio frequency electrosurgical waveform from an output stage to one or more pairs of electrodes; passing the at least one radio frequency electrosurgical waveform to the pair of electrodes through a bridge rectifier; transforming at least a portion of the at least one radio frequency electrosurgical waveform into direct current; and measuring current of the direct current.

### BRIEF DESCRIPTION OF THE DRAWINGS

Various embodiments of the present disclosure are described herein with reference to the drawings wherein:
Fig. 1 is a schematic block diagram of an electrosurgical system in accordance with the present disclosure;
Fig. 2 is a schematic diagram of the electrosurgical generator of Fig. 1 in accordance with the present disclosure;
Fig. 3 is a schematic diagram of an isolated current in accordance with the present disclosure; and

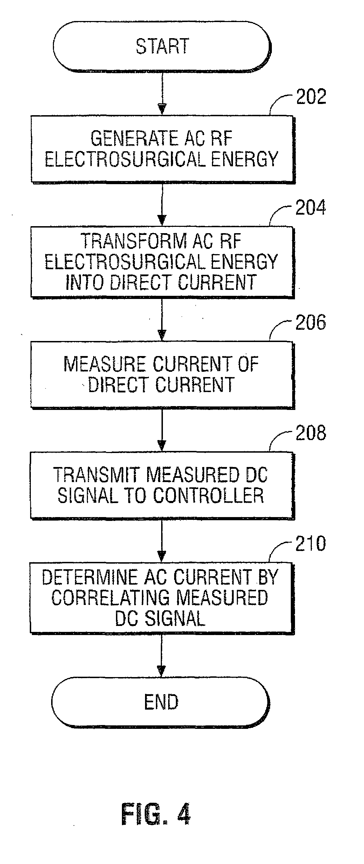

Fig. 4 is a flow chart of a method in accordance with the present disclosure.

### DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

A generator according to the present disclosure can perform monopolar and/or bipolar electrosurgical procedures, including vessel sealing procedures. The generator may include a plurality of outputs for interfacing with various electrosurgical instruments (e.g., a monopolar instrument, return electrode, bipolar electrosurgical forceps, footswitch, etc.). Further, the generator includes electronic circuitry configured to generate radio frequency energy specifically suited for various electrosurgical modes (e.g., cutting, blending, division, etc.) and procedures (e.g., monopolar, bipolar, vessel sealing). In embodiments, the generator may be embedded, integrated or otherwise coupled to the electrosurgical instruments providing for an all-in-one electrosurgical apparatus.

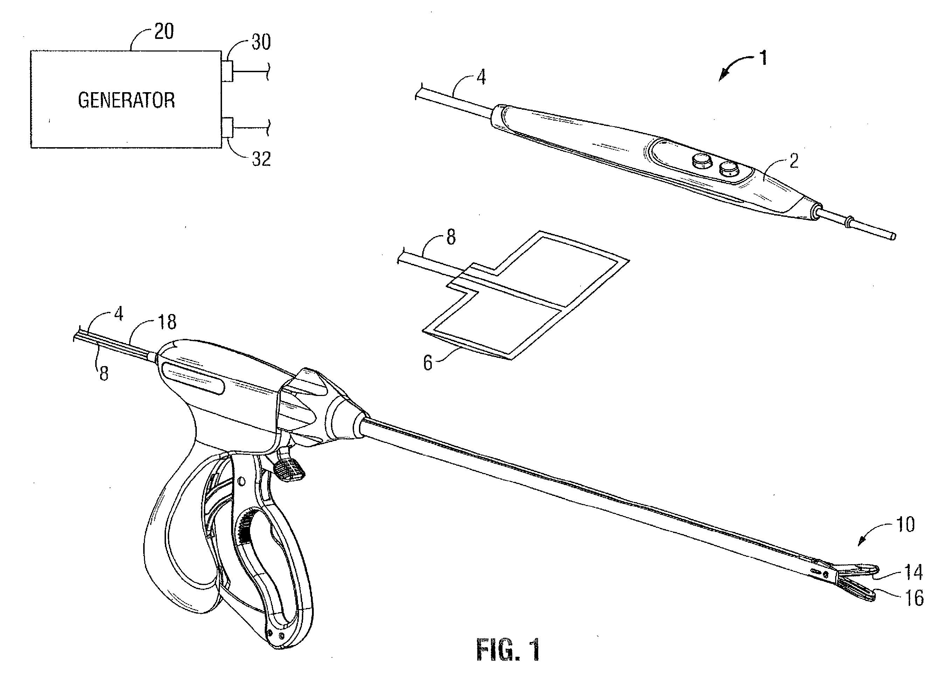

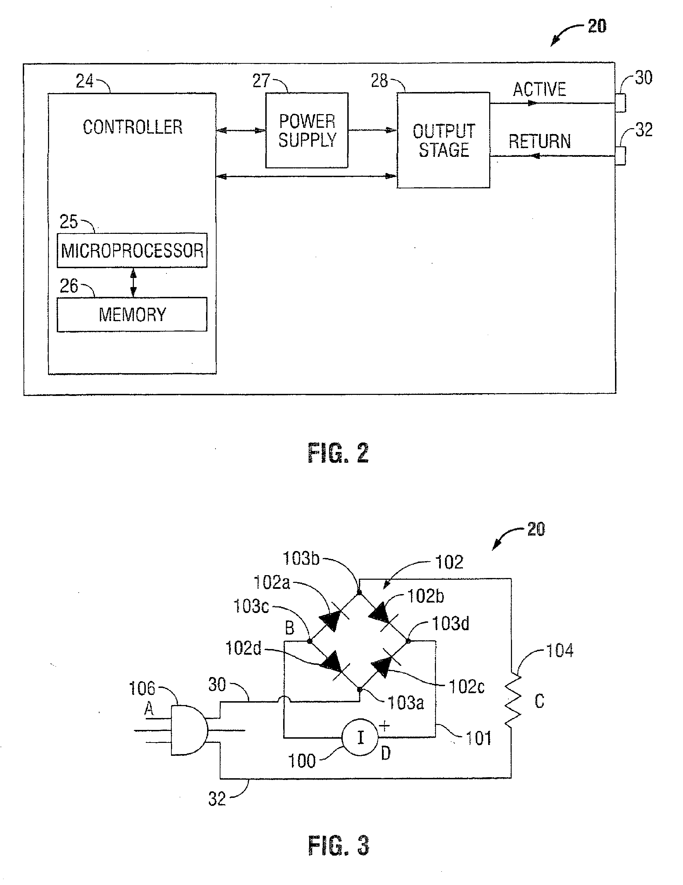

Fig. 1 is a schematic illustration of a bipolar and monopolar electrosurgical system 1 according to the present disclosure. The system 1 may include one or more monopolar electrosurgical instruments 2 having one or more electrodes (e.g., electrosurgical cutting probe, ablation electrode(s), etc.) for treating tissue of a patient. Electrosurgical energy is supplied to the instrument 2 by a generator 20 via a supply line 4 that is connected to an active terminal 30 (Fig. 3) of the generator 20, allowing the instrument 2 to coagulate, ablate and/or otherwise treat tissue. The energy is returned to the generator 20 through a return electrode 6 via a return line 8 at a return terminal 32 (Fig. 3) of the generator 20. The system 1 may include a plurality of return electrodes 6 that are disposed on a patient to minimize the chances of tissue damage by maximizing the overall contact area with the patient. In addition, the generator 20 and the return electrode 6 may be configured for monitoring so-called "tissue-to-patient" contact to insure that sufficient contact exists therebetween to further minimize chances of tissue damage.

The system 1 may also include a bipolar electrosurgical forceps 10 having one or more electrodes for treating tissue of a patient. The electrosurgical forceps 10 includes opposing jaw members having one or more active electrodes 14 and a return electrode 16 disposed therein. The active electrode 14 and the return electrode 16 are connected to the generator 20 through cable 18 that includes the supply and return lines 4, 8 coupled to the active and return terminals 30, 32, respectively. The electrosurgical forceps 10 is coupled to the generator 20 at a connector having connections to the active and return terminals 30 and 32 (e.g., pins) via a plug disposed at the end of the cable 18, wherein the plug includes contacts from the supply and return lines 4, 8.

In embodiments, the generator 20 may be configured as a portable generator and may be housed detachably or otherwise within the electrosurgical instrument 2 or the electrosurgical forceps 10. The generator 20 may be any suitable type (e.g., electrosurgical, microwave, etc.) and may include a plurality of suitable input controls (e.g., buttons, activators, switches, touch screen, etc.) for controlling the generator 20. The controls may be disposed on the generator 20 or on the electrosurgical instrument 2 or the electrosurgical forceps 10.

Fig. 2 shows a schematic block diagram of the generator 20 configured to output electrosurgical energy. In another embodiment, the generator 20 may be configured to output other types of energy such as, microwave, laser, etc. to power various other tissue treatment devices, such as microwave antennas, ultrasonic forceps, lasers, resistive heating electrodes, etc. The generator 20 includes a controller 24, a power supply 27, and an output stage 28. The power supply 27 may be a direct current high voltage power supply and is connected to an AC source (e.g., electrical wall outlet) and provides high voltage DC power to an output stage 28, which then converts high voltage DC power into treatment energy (e.g., ultrasonic, electrosurgical or microwave) and delivers the energy to the active terminal 30. The energy is returned thereto via the return terminal 32. The output stage 28 is configured to operate in a plurality of modes, during which the generator 20 outputs corresponding waveform having specific duty cycles, peak voltages, crest factors, etc. In another embodiment, the generator 20 may be based on other types of suitable power supply topologies.

The controller 24 includes a microprocessor 25 operably connected to a memory 26, which may be volatile type memory (e.g., RAM) and/or non-volatile type memory (e.g., flash media, disk media, etc.). The microprocessor 25 includes an output port that is operably connected to the power supply 27 and/or output stage 28 allowing the microprocessor 25 to control the output of the generator 20 according to either open and/or closed control loop schemes. Those skilled in the art will appreciate that the microprocessor 25 may be substituted by any logic processor (e.g., control circuit) adapted to perform the calculations discussed herein.

A closed loop control scheme is a feedback control loop, in which a plurality of sensors measure a variety of tissue and energy properties (e.g., tissue impedance, tissue temperature, output power, current and/or voltage, etc.), and provide feedback to the controller 24. Such sensors are within the purview of those skilled in the art. The controller 24 then signals the power supply 27 and/or output stage 28, which then adjusts the DC and/or power supply, respectively. The controller 24 also receives input signals from the input controls of the generator 20, the instrument 2 or forceps 10. The controller 24 utilizes the input signals to adjust power outputted by the generator 20 and/or performs other control functions thereon.

Systems and methods for sensing alternating current may include very low resistive elements, such as shunt resistors, capacitors, current transformers and the like. Some of the current sensor designs are limited by safety requirements associated with electrosurgical procedures. In particular, in electrosurgical applications, voltage isolation barrier are desired or required to protect sensing circuitry from large voltages or to reduces coupled noise and interference. This prevents the use of shunt resistors and capacitors as detailed above for sensing purposes without the use of additional isolation circuit components, such as optical couplers and the like, to bridge the isolation barrier.

In one embodiment, current transformer or other sensors may be used. Current transformers may be of various sizes and varying efficiencies, particularly at high frequencies However, current transformers do not lend themselves well to miniaturization. Accordingly, a Hall Effect device or optical couplers that have a very small footprint may be used. However, such devices suffer from a low frequency response. For AC current signals above about 100 kHz, Hall effect devices are not particularly compatible, especially in higher current applications when used to sense current directly. Due to these limitations for frequencies from about 100 kHz to about 2 MHz, in embodiments from about 200 kHz to about 1 MHz, neither the current sense transformers nor the Hall effect sensors are useful when miniaturization is required.

The present disclosure overcomes the issues involved with miniaturized current sensors and provides for an isolated current sensor that is miniaturized and operates at frequencies from about 100 kHz to about 2 MHz, in embodiments from about 200 kHz to about 1 MHz. As shown in Fig. 3, the generator 20 includes an isolated current sensor 100 that is coupled to a bridge rectifier 102 that is disposed between a load 104 and a power source 106. The power source 106 represents the energy output of the output stage 28 through the active and return terminals 30 and 32. The load 104 may be any resistive load into which the energy of the power source 106 is transmitted to, such as tissue being grasped by the electrosurgical forceps 10.

The bridge rectifier 102 is shown as a full-wave diode bridge of four diodes 102a, 102b, 102c, 102d including first and second input terminals 103a and 103b, with the first input terminal 103a coupled to the power source 106 and the second input terminal 103b coupled to the load 104 through the active terminal 30. The bridge rectifier 102 also includes first and second output terminals 103c and 103d coupled to the isolated current sensor 100 through a coupling member 101, which may be any conductive element interconnecting the first and second output terminals 103c and 103d. In embodiments, the diodes 102a, 102b, 102c, 102d may be any suitable circuit element or configuration, such as an ideal diode, configured to conduct current substantially in one direction.

During operation, the bridge rectifier 102 provides a DC current to the isolated current sensor 100 without altering the AC characteristics of the AC (e.g., RF) energy transmitted from the power source 106 to the load 104. In particular, the bridge rectifier 102 allows the AC current to pass therethrough and to convert a portion of the AC current from the power source 106 into DC current that is then sensed by the isolated current sensor 100 while the load 104 is still fed by the AC current.

Since AC current operates in a push-pull manner, during the push or positive cycle, the AC current flows from the active terminal 30 to the return terminal 32, namely, the AC current flows through the diode 102c to the isolated current sensor 100 and then through the diode 102a to the load 104 and the return terminal 32. During the pull or negative cycle, the AC current flows from the return terminal 32 to the active terminal 30, namely, the AC current flows through the diode 102b to the isolated current sensor 100 and then through the diode 102d back to the power source 106.

The pairs of diodes 102a and 102c and 102b and 102d transform the AC current into DC current, which is then measured by the isolated current sensor 100. In embodiments, the isolated current sensor 100 may be a Hall Effect sensor or any other lower frequency sensing device that does not alter the AC characteristics of the power source 106 and the load 104. The isolated current sensor 100 measures the variations in the magnetic field caused by the changes in the current transmitted through the coupling member 101. In embodiments, the isolated current sensor 100 is disposed in proximity to the coupling member 101 based on the sensitivity of the Hall Effect sensor, obviating the need for physical contact therebetween. Thus, the isolated current sensor 100 measures a converted DC current without interrupting the circuit, thereby being transparent to the power source 106 and the load 104. The current sensor 100 is coupled to the controller 24 and supplies a signal corresponding to the measured amplitude of the direct current thereto. The controller 24 may process the current signal by using any type of a processing function that correlates the AC output of the power source 106 with the converted DC current. The measured DC current reflective of the output AC current may be used in a variety of ways, including algorithm control, dosage error checks, and other output verification.

Fig, 4 shows a flow chart of a method 200 according to the present disclosure. The method includes a step 202 during which the power source 106, e.g., the output stage 28, outputs AC RF therapeutic electrosurgical energy to the load 104, e.g., patient tissue. In step 204, as the energy is transmitted to the load 104, the bridge rectifier 102 transforms a portion of the AC current into DC, which is then transmitted to the isolated current sensor 100. In step 206, the isolated current sensor 100 measures the variations in the DC current transmitted through the coupling member 101. In step 208, the current sensor 100 transmits the DC current signal to the controller 24, which in step 210, then correlates the signal to the AC current output to determine actual AC current output.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. An electrosurgical generator comprising:
an output stage (28) configured to generate at least one radio frequency electrosurgical waveform;
a bridge rectifier (102) coupled to the output stage (28) and configured to pass-through the at least one radio frequency electrosurgical waveform from the output stage to patient tissue as a load (104) and to transform at least a portion of the at least one radio frequency electrosurgical waveform into direct current; and
an isolated current sensor (100) coupled to the bridge rectifier and configured to measure amplitude of the direct current.

2. The electrosurgical generator according to claim 1, wherein the isolated current sensor (100) is a Hall Effect sensor.

3. The electrosurgical generator according to claim 1 or 2, wherein the bridge rectifier (102) includes:
first and second intput terminals (103a, 103b) coupled between the output stage (28) and the load (104).

4. The electrosurgical generator according to anyone of claims 1 to 3, wherein the bridge rectifier (102) includes:
first and second output terminals (103c, 103d) interconnected by at least one coupling member

5. The electrosurgical generator according to claim 4, wherein the isolated current sensor (100) is a Hall Effect sensor disposed in proximity to the at least one coupling member.

6. The electrosurgical generator according to anyone of claims 1 to 5, further comprising a controller (24) coupled to the isolated current sensor (100), the controller (24) configured to determine radio frequency current of the at least one radio frequency electrosurgical waveform based on the direct current measured by the isolated current sensor (100).

7. An electrosurgical system comprising:
at least one pair of electrodes (30, 32) coupled to a load (104);
an output stage (28) coupled to the at least one pair of electrodes (30, 32), the output stage (28) configured to generate at least one radio frequency electrosurgical waveform;
a bridge rectifier (102) coupled to the output stage (28) and configured to pass-through the at least one radio frequency electrosurgical waveform from the output stage to patient tissue as the load (104) and to transform at least a portion of the at least one radio frequency electrosurgical waveform into direct current; and
an isolated current sensor (100) coupled to the bridge rectifier and configured to measure amplitude of the direct current.

8. The electrosurgical system according to claim 7, wherein the isolated current sensor (100) is a Hall Effect sensor.

9. The electrosurgical system according to claim 7 or 8, wherein the bridge rectifier (102) includes:
first and second input terminals (103a, 103b) coupled between the output stage and the load (104).

10. The electrosurgical system according to anyone of claims 7 to 9, wherein the bridge rectifier (102) includes:
first and second output terminals (103c, 103d) interconnected by at least one coupling member.

11. The electrosurgical system according to claim 10,
wherein the isolated current sensor (100) is a Hall Effect sensor disposed in proximity to the at least one coupling member.

12. The electrosurgical system according to anyone of claims 7 to 11, further comprising a controller (24) coupled to the isolated current sensor (100), the controller (24) configured to determine radio frequency current of the at least one radio frequency electrosurgical waveform based on the direct current measured by the isolated current sensor (100).

## Patentansprüche

1. Elektrochirurgischer Generator, umfassend:
- eine Ausgangsstufe (28), die konfiguriert ist, um mindestens eine elektrochirurgische Hochfrequenz-Wellenform zu erzeugen,
- einen Brückengleichrichter (102), der mit der Ausgangsstufe (28) gekoppelt ist und konfiguriert ist, um die mindestens eine elektrochirurgische Hochfrequenz-Wellenform von der Ausgangsstufe zu einem Patientengewebe als Last (104) durchzugeben und um mindestens einen Teil der mindestens einen elektrochirurgischen Hochfrequenz-Wellenform in Gleichstrom umzuwandeln; und
- einen isolierten Stromsensor (100), der mit dem Brückengleichrichter gekoppelt ist und konfiguriert ist, um die Amplitude des Gleichstroms zu messen.

2. Elektrochirurgischer Generator nach Anspruch 1, wobei der isolierte Stromsensor (100) ein Hall-Effektsensor ist.

3. Elektrochirurgischer Generator nach Anspruch 1 oder 2, wobei der Brückengleichrichter (102) Folgendes umfasst:
- erste und zweite Eingangsanschlüsse (103a, 103b), die zwischen der Ausgangsstufe (28) und der Last (104) gekoppelt sind.

4. Elektrochirurgischer Generator nach einem der Ansprüche 1 bis 3, wobei der Brückengleichrichter (102) Folgendes umfasst:
- erste und zweite Ausgangsanschlüsse (103c, 103d), die durch mindestens ein Kopplungselement zusammengeschaltet sind.

5. Elektrochirurgischer Generator nach Anspruch 4, wobei der isolierte Stromsensor (100) ein Hall-Effektsensor ist, der in der Nähe des mindestens einen Kopplungselements angeordnet ist.

6. Elektrochirurgischer Generator nach einem der Ansprüche 1 bis 5, ferner umfassend einen Controller (24), der mit dem isolierten Stromsensor (100) gekoppelt ist, wobei der Controller (24) konfiguriert ist, um einen Hochfrequenzstrom der mindestens einen elektrochirurgischen Hochfrequenz-Wellenform basierend auf dem Gleichstrom, der von dem isolierten Stromsensor (100) gemessen wird, zu bestimmen.

7. Elektrochirurgisches System, umfassend:
- mindestens ein Paar Elektroden (30, 32), die mit einer Last (104) gekoppelt sind;
- eine Ausgangsstufe (28), die mit dem mindestens einen Paar Elektroden (30, 32) gekoppelt ist, wobei die Ausgangsstufe (28) konfiguriert ist, um mindestens eine elektrochirurgische Hochfrequenz-Wellenform zu erzeugen;
- einen Brückengleichrichter (102), der mit der Ausgangsstufe (28) gekoppelt ist und konfiguriert ist, um die mindestens eine elektrochirurgische Hochfrequenz-Wellenform von der Ausgangsstufe zu einem Patientengewebe als Last (104) durchzugeben und um mindestens einen Teil der mindestens einen elektrochirurgischen Hochfrequenz-Wellenform in Gleichstrom umzuwandeln; und
- einen isolierten Stromsensor (100), der mit dem Brückengleichrichter gekoppelt ist und konfiguriert ist, um die Amplitude des Gleichstroms zu messen.

8. Elektrochirurgisches System nach Anspruch 7, wobei der isolierte Stromsensor (100) ein Hall-Effektsensor ist.

9. Elektrochirurgisches System nach Anspruch 7 oder 8, wobei der Brückengleichrichter (102) Folgendes umfasst:
- erste und zweite Eingangsanschlüsse (103a, 103b), die zwischen der Ausgangsstufe (28) und der Last (104) gekoppelt sind.

10. Elektrochirurgisches System nach einem der Ansprüche 7 bis 9, wobei der Brückengleichrichter (102) Folgendes umfasst:
- erste und zweite Ausgangsanschlüsse (103c, 103d), die durch mindestens ein Kopplungselement zusammengeschaltet sind.

11. Elektrochirurgisches System nach Anspruch 10, wobei der isolierte Stromsensor (100) ein Hall-Effektsensor ist, der in der Nähe des mindestens einen Kopplungselements angeordnet ist.

12. Elektrochirurgisches System nach einem der Ansprüche 7 bis 11, ferner umfassend einen Controller (24), der mit dem isolierten Stromsensor (100) gekoppelt ist, wobei der Controller (24) konfiguriert ist, um einen Hochfrequenzstrom der mindestens einen elektrochirurgischen Hochfrequenz-Wellenform basierend auf dem Gleichstrom, der von dem isolierten Stromsensor (100) gemessen wird, zu bestimmen.

## Revendications

1. Générateur électro-chirurgical, comprenant :
un étage de sortie (28) configuré pour produire au moins une forme d'onde électro-chirurgicale de radiofréquence ;
un pont redresseur (102) couplé à l'étage de sortie (28) et configuré pour passer à travers la au moins une forme d'onde électro-chirurgical de radiofréquence depuis l'étage de sortie au tissu d'un patient comme une charge (104) et pour transformer au moins une portion de la au moins une forme d'onde électro-chirurgicale de radiofréquence en courant continu ; et
un capteur de courant isolé (100) couplé au pont redresseur et configuré pour mesurer l'amplitude du courant continu.

2. Générateur électro-chirurgical selon la revendication 1, dans lequel le capteur de courant isolé (100) est un capteur à effet Hall.

3. Générateur électro-chirurgical selon la revendication 1 ou 2, dans lequel le pont redresseur (102) comprend :
des première et deuxième bornes d'entrée (103a, 103b) couplées entre l'étage de sortie (28) et la charge (104).

4. Générateur électro-chirurgical selon l'une quelconque des revendications 1 à 3, dans lequel le pont redresseur (102) comprend :
des première et deuxième bornes de sortie (103c, 103d) interconnectées par au moins un élément de couplage.

5. Générateur électro-chirurgical selon la revendication 4, dans lequel le capteur de courant isolé (100) est un capteur à effet Hall disposé à proximité du au moins un élément de couplage.

6. Générateur électro-chirurgical selon l'une quelconque des revendications 1 à 5, comprenant en outre un dispositif de commande (24) couplé au capteur de courant isolé (100), le dispositif de commande (24) étant configuré pour déterminer un courant de radiofréquence de la au moins une forme d'onde électro-chirurgicale de radiofréquence basé sur le courant continu mesuré par le capteur de courant isolé (100).

7. Système électro-chirurgical comprenant :
au moins une paire d'électrodes (30, 32) couplées à une charge (104) ;
un étage de sortie (28) couplé à la au moins une paire d'électrodes (30, 32), l'étage de sortie (28) étant configuré pour produire au moins une forme d'onde électro-chirurgicale de radiofréquence ;
un pont redresseur (102) couplé à l'étage de sortie (28) et configuré pour passer à travers la au moins une forme d'onde électro-chirurgicale de radiofréquence depuis l'étage de sortie au tissu d'un patient comme charge (104) et pour transformer au moins une portion de la au moins une forme d'onde électro-chirurgicale de radiofréquence en courant continu ; et
un capteur de courant isolé (100) couplé au pont redresseur et configuré pour mesurer l'amplitude du courant continu.

8. Système électro-chirurgical selon la revendication 7, dans lequel le capteur de courant isolé (100) est un capteur à effet Hall.

9. Système électro-chirurgical selon la revendication 7 ou 8, dans lequel le pont redresseur (102) comprend :
des première et deuxième bornes d'entrée (103a, 103b) couplées entre l'étage de sortie et la charge (104).

10. Système électro-chirurgical selon l'une quelconque des revendications 7 à 9, dans lequel le pont redresseur (102) comprend :
des première et deuxième bornes de sortie (103c, 103d) interconnectées par au moins un élément de couplage.

11. Système électro-chirurgical selon la revendication 10, dans lequel le capteur de courant isolé (100) est un capteur à effet Hall disposé à proximité du au moins un élément de couplage.

12. Système électro-chirurgical selon l'une quelconque des revendications 7 à 11, comprenant en outre un dispositif de commande (24) couplé au capteur de courant isolé (100), le dispositif de commande (24) étant configuré pour déterminer le courant de radiofréquence de la au moins une forme d'onde électro-chirurgicale de radiofréquence basé sur le courant continu mesuré par le capteur de courant isolé (100).
